(19) Europäisches Patentamt
European Patent Office
Office européen des brevets

(11) Publication number: 0 252 989 A1

(12) **EUROPEAN PATENT APPLICATION**

published in accordance with Art. 158(3) EPC

(21) Application number: 87900289.7

(51) Int. Cl.⁴: C 07 H 19/073

(22) Date of filing: 26.12.86

Data of the international application taken as a basis:

(86) International application number: PCT/JP 86/00660

(87) International publication number: WO 87/03881 (02.07.87 87/14)

(30) Priority: 26.12.85 JP 294977/85

(43) Date of publication of application: 20.01.88 Bulletin 88/3

(84) Designated Contracting States: CH DE FR GB IT LI NL

(71) Applicant: TAIHO PHARMACEUTICAL COMPANY, LIMITED, 27, Kandanishiki-cho 1-chome Chiyoda-ku, Tokyo 101 (JP)

(72) Inventor: UEDA, Toru 402-21, Godo-Shukusha, 3-jo 10-chome Miyanomori, Chuo-ku, Sapporo-shi Hokkaido 064 (JP)
Inventor: YASUMOTO, Mitsugi, 2-8-19, Maehara Honjo-shi, Saitama 367 (JP)
Inventor: YAMASHITA, Junichi, 11-jo 7-chome Shinkotoni, Kita-ku Sapporo-shi, Hokkaido 001 (JP)
Inventor: MATSUMOTO, Hiroshi, 3328-1, Honjo-shi, Saitama 367 (JP)
Inventor: TADA, Yukio, 3-7-30, Hinode Honjo-shi, Saitama 367 (JP)
Inventor: KOBAYASHI, Kazuhiro, 4-7-20, Higashidai Honjo-shi, Saitama 367 (JP)
Inventor: NOGUCHI, Kazuharu, 1128-2, Aza Kasagayato Kitabori Honjo-shi, Saitama 367 (JP)

(74) Representative: Wotherspoon, Graham et al, FITZPATRICKS 4 West Regent Street, Glasgow G2 1RS Scotland (GB)

(54) **2'-DEOXY-5-TRIFLUOROMETHYLURIDINE DERIVATIVES AND PROCESS FOR THEIR PREPARATION.**

(57) 2'-deoxy-5-trifluoromethyluridine derivatives represented by general formula (I), (wherein Tr represents a triphenylmethyl group and R represents a lower alkyl group or a benzyl group) and a process for their preparation. The derivatives are prepared by subjecting a 2'-deoxy-5 halogenuridine derivative represented by general formula (II), (wherein W represents an iodine atom or a bromine atom, Tr represents a triphenylmethyl group, and R represents a lower alkyl group or a benzyl group) to trifluoromethylation reaction with halogenotrifluoromethane in the presence of metallic copper.

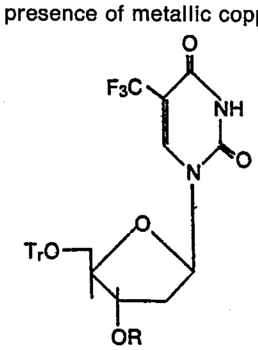

(I)

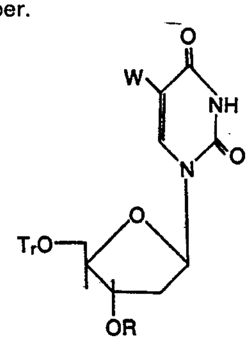

(II)

EP 0 252 989 A1

# 2'-DEOXY-5-TRIFLUOROMETHYLURIDINE DERIVATIVES AND PROCESS FOR PREPARING THE SAME

## Industrial Application of the Invention

The present invention relates to novel 2'-deoxy-5-trifluoromethyluridine derivatives and to a process for preparing the same. The 2'-deoxy-5-trifluoromethyluridine derivatives of the present invention are useful as intermediates for preparing 2'-deoxy-5-trifluoromethyluridine derivatives (Japanese Unexamined Patent Publication No. 216899/1984) having an excellent anticancer activity and represented by the formula

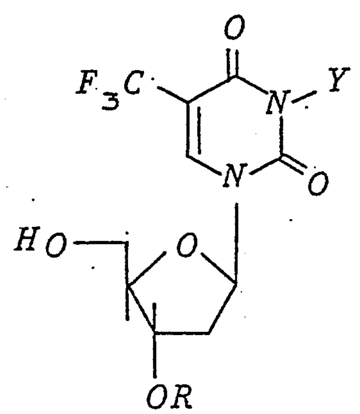

( I )

wherein R is lower alkyl or benzyl and Y is hydrogen or benzoyl.

## Prior Art and Its Problems

The 2'-deoxy-5-trifluoromethyluridine derivative

of the formula (I) has been conventionally prepared by reacting a 2'-deoxy-5-trifluoromethyluridine as a starting material with a benzoic acid halide to obtain a 3-benzoyl-2'-deoxy-5-trifluoromethyluridine represented by the formula

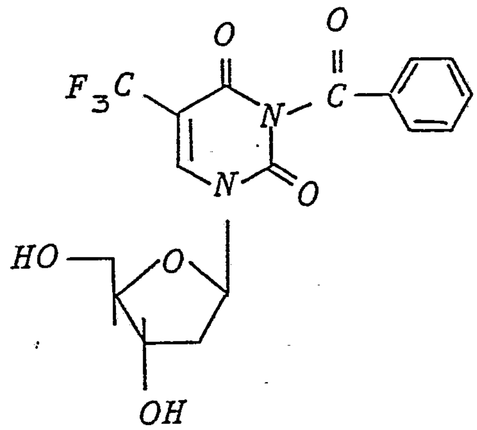

( II )

and reacting the compound of the formula (II) thus obtained with an alkyl halide or benzyl halide in the presence of a metallic oxide catalyst, e.g., silver oxide to produce a monoether derivative of 3-benzoyl-2'-deoxy-5-trifluoromethyluridine represented by the formula

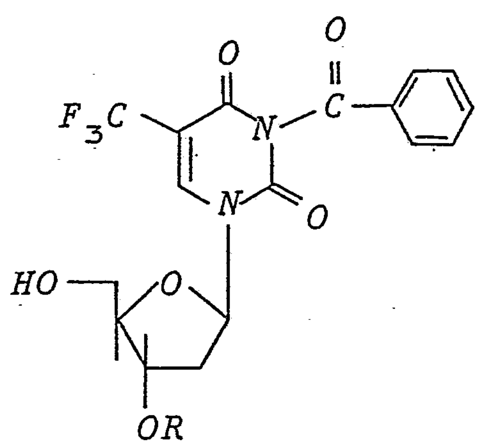

( I' )

wherein R is as defined above, the compound of the formula (I') thus obtained being optionally acted on by an acid or an alkali to undergo debenzoylation reaction (Japanese Unexamined Patent Publication No.216899/1984).

However, the conventional process stated above has the problem of giving the compound of the formula (I') from the compound of the formula (II) in a very low yield of 10% or less because the reaction process for obtaining the compound of the formula (I') produces by-products such as a compound represented by the formula

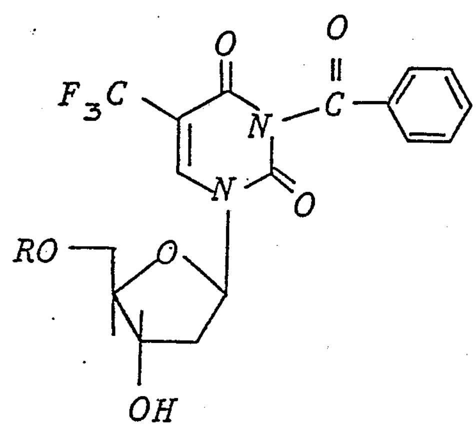

( III )

wherein R is as defined above. The process stated above has further problems that the process is cumbersome to carry out because it needs to use a column chromatography or the like for separating the compound of the formula (I') from the compound of the formula (III) and that the silver oxide or the like to be used as a catalyst is expensive. Accordingly the foregoing conventional process

has been inadequate as an industrial producing process.

## Measure for Solution of the Problems

The present inventors conducted intensive research to develop a suitable industrial process for preparing the compound of the formula (I) free of said problems, and found a novel intermediate for producing the compound of the formula (I) which intermediate is able to achieve this object. The present invention has been accomplished based on this novel finding.

## Disclosure of the Invention

The present invention provides a 2'-deoxy-5-trifluoromethyluridine derivative represented by the formula

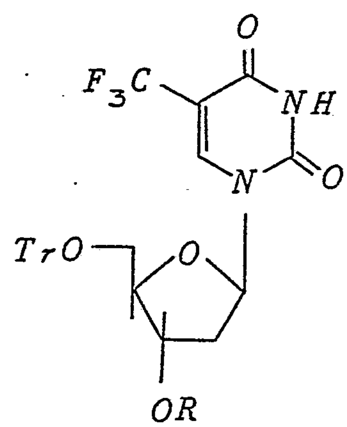

( IV )

wherein Tr is triphenylmethyl and R is lower alkyl or benzyl, and a process to be described later for preparing the same.

The compound of the present invention

represented by the formula (IV) can be easily made into the compound of the formula (I) in an extremely high yield. In making the compound of the formula (IV) into the compound of the formula (I), neither a cumbersome separating procedure nor an expensive catalyst is required. Accordingly the compound of the invention is extremely useful for suitable industrial manufacture of the compound of the formula (I).

Examples of lower alkyl groups represented by R in the formula (IV) are alkyl groups having 1 to 6 carbon atoms, such as methyl, ethyl, propyl, butyl, hexyl and the like.

The compound of the formula (IV) according to the present invention can be prepared, for example, according to Reaction Scheme A shown below.

<Reaction Scheme A>

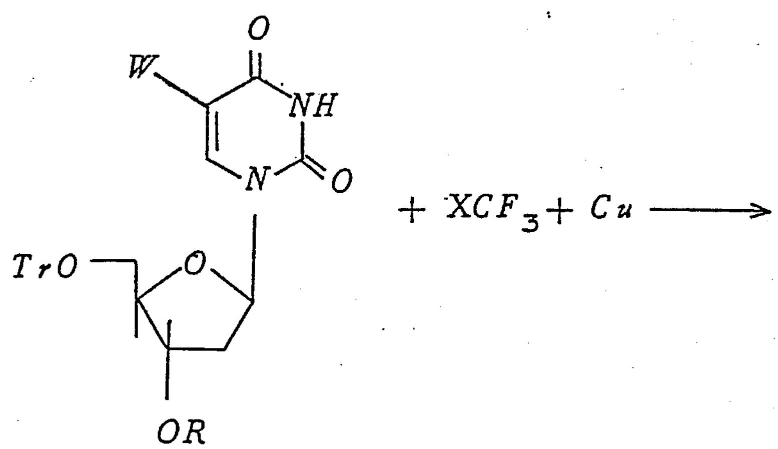


(IV)

(V)

wherein Tr and R are as defined above, and W and X are iodine or bromine.

The compound of the formula (IV) can be produced by subjecting a 2'-deoxy-5-halogenouridine derivative of the formula (V) to trifluoromethylation reaction with a halogenotrifluoromethane in the presence of a metallic copper. The compound of the formula (V) to be used in this reaction is a novel compound and can be prepared, for example, in a manner to be described later in Reference Examples 1 and 2.

The trifluoromethylation reaction is carried out usually with a suitable solvent or without a solvent in the presence of a halogenotrifluoromethane and a metallic copper within a suitable closed vessel, e.g. autoclave, sealed tube or the like.

The kind of solvents to be used for the reaction is not specifically limited insofar as the solvent does not affect the reaction. Specific examples thereof are aprotic polar solvents such as acetonitrile, dimethylformamide, dimethylacetoamide, hexamethylphosphoric triamide (HMPA) and the like; amines such as triethylamine, pyridine, dimethylaniline, dimethylaminopyridine and the like; ethers such as tetrahydrofuran, dioxane and the like; ketones such as 3-pentanone and the like; etc. These solvents can be used

singly or in mixture.

The amount of halogenotrifluoromethane, which is variable with the kind of halogen atom, is definitely indeterminable but usually ranges from about 1 to about 100 moles, preferably about 2 to about 50 moles, per mole of the compound of the formula (V). The amount of metallic copper, although variable with the kind of halogenotrifluoromethane and definitely indeterminable, ranges from about 1 to about 100 moles, preferably about 2 to about 80 moles, per mole of the the compound of the formula (V). While variable with the kind of halogenotrifluoromethane, the reaction temperature is in the range of room temperature to about 200°C, preferably 50 to 160°C. The reaction time is variable with the kind of halogenotrifluoromethane and the reaction temperature, but 24 hours or less of the reaction is generally sufficient.

Prior to the trifluoromethylation reaction, a halogenotrifluoromethane may be reacted with a metallic copper to give a reaction product, which is then reacted with the compound of the formula (V). In the reaction of the reaction product of halogenotrifluoromethane and metallic copper with the compound of the formula (V), preferred reaction temperature is between room temperature and about 100°C, and the reaction time of 15 hours or less

is generally sufficient although variable with the reaction temperature.

The compound of the formula (IV) of the present invention thus obtained can be made into the compound of the formula (I), for example, according to Reaction Scheme B shown below.

<Reaction Scheme B>

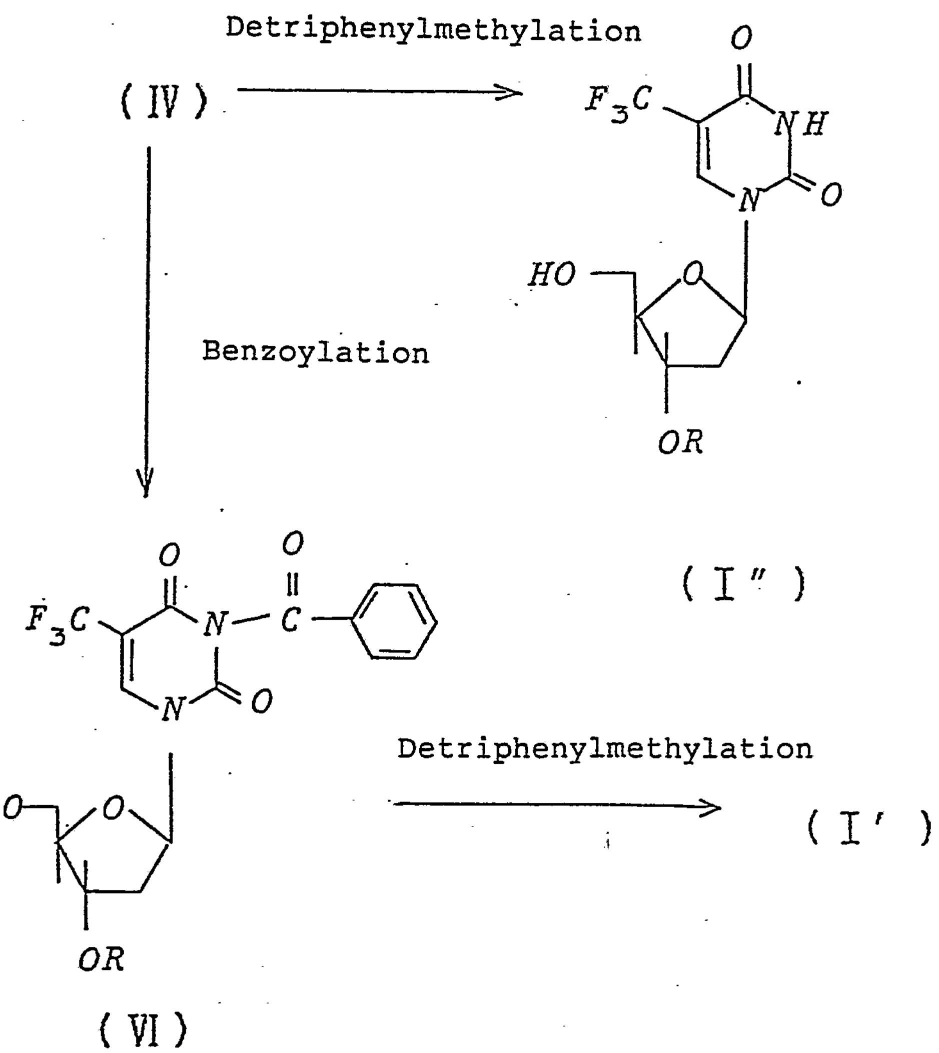


( IV )

( I″ )

( VI )

( I′ )

wherein Tr and R are as defined above.

The compound of the formula (IV) according to the present invention is subjected to detriphenylmethylation under the conditions to be described later in Reference Examples, giving a compound of the formula (I''), or alternatively the compound of the formula (IV) of the present invention is benzoylated in the presence of a base to produce a compound of the formula (VI), which is subsequently subjected to detriphenylmethylation, affording a compound of the formula (I'). The procedures stated above are all made to proceed substantially quantitatively. The reaction product as it is which results from the trifluoromethylation reaction, to say nothing of the compound separated from the reaction product, is also available as the compound of the formula (IV) of the present invention to be used in Reaction Scheme B.

The compounds prepared by the processes as described above can be purified by conventional separation methods such as recrystallization or the like.

Examples

Given below are Examples and Reference Examples to describe the present invention in detail. Reference Example 4 is illustrative of a process in which the detriphenylmethylation reaction is conducted using the

compound of the present invention without isolation to make a useful compound which is separated from the reaction product by a simplified procedure.

Reference Example 1

Preparation of 3'-O-ethyl-5'-O-triphenylmethyl-2'-deoxy-5-iodouridine

Tetrahydrofuran (200 ml) was added to 23.9 g (0.04 mole) of 5'-O-triphenylmethyl-2'-deoxy-5-iodouridine and 2.4 g (0.1 mole) of sodium hydride. The mixture was stirred at room temperature for 0.5 hour and then 9.4 g (0.06 mole) of ethyl iodide was added to the reaction mixture. The mixture was stirred at 50°C for 6 hours. The solvent was distilled off under reduced pressure and water was added to the residue. The mixture was extracted with ethyl acetate. After the extract was washed with water, the solvent was distilled off under reduced pressure. The residue was subjected to silica gel column chromatography, whereby 20.5 g of 3'-O-ethyl-5'-O-triphenylmethyl-2'-deoxy-5-iodouridine was obtained from the chloroform eluate (82% yield).

Nuclear magnetic resonance spectrum

(TMS internal standard, in DMSO-$d_6$)

δ ppm;

11.70 (1H, s, NH), 8.00 (1H, s, $H^6$), 7.5-7.1 (15H, m, aromatic H), 6.00 (1H, t, $H^{1'}$), 4.1-3.9 (2H, m, $H^{3'}$

and $H^{4'}$), 3.38 (2H, q, $-OC\underline{H_2}CH_3$), 3.1-3.4 (2H, m, $H^{5'}$), 2.28 (1H, broad t, $H^{2'}$), 1.07 (3H, t, $-OCH_2C\underline{H_3}$)

Reference Example 2

Preparation of 3'-O-benzyl-5'-O-triphenylmethyl-2'-deoxy-5-iodouridine

Tetrahydrofuran (250 ml) was added to 29.8 g (0.05 mole) of 5'-O-triphenylmethyl-2'-deoxy-5-iodouridine and 3 g (0.125 mole) of sodium hydride. After the mixture was stirred at room temperature for 0.5 hour, 12.8 g (0.075 mole) of benzyl bromide was added to the mixture and the mixture was stirred at 50°C for 4 hours. Methanol was added to the reaction mixture to decompose the excess sodium hydride. The solvent was distilled off under reduced pressure. Water was added to the residue, and the mixture was extracted with ethyl acetate. The extract was washed with water after which the solvent was evaporated off under reduced pressure. The residue was recrystallized from dichloromethane-methanol, giving 30.8 g of 3'-O-benzyl-5'-O-triphenylmethyl-2'-deoxy-5-iodouridine (90% yield). M.p. 189-191°C.

Reference Example 3

Preparation of 3'-O-benzyl-2'-deoxy-5-trifluoromethyl-uridine

A 20 ml quantity of 2% hydrochloric acid-methanol was added to 3.14 g (5 mmoles) of 3'-O-benzyl-5'-O-triphenylmethyl-2'-deoxy-5-trifluoromethyluridine. The mixture was stirred at room temperature for 3 hours. The reaction mixture was neutralized with triethylamine and the solvent was distilled off under reduced pressure. After addition of water, the mixture was extracted with methylene chloride. The extract was washed with water, followed by evaporation of the solvent under reduced pressure. Methanol was added to the residue after which the precipitated crystals were removed by filtration. The mother liquor was concentrated under reduced pressure, followed by addition of chloroform. The crystals precipitated were collected by filtration, giving 1.78 g of 3'-O-benzyl-2'-deoxy-5-trifluoromethyluridine having a melting point of 157 to 158°C (92% yield).

Reference Example 4

Preparation of 3'-O-ethyl-2'-deoxy-5-trifluoromethyl-uridine

To 3.0 g (0.047 mole) of powdery copper was added 24 ml of dimethylformamide/pyridine (3 : 1). Into the mixture was introduced 10.8 g (0.072 mole) of trifluoromethane bromide and the mixture was stirred in a sealed tube at 140°C for 5 hours. After cooling, 2.25 g (3.6 mmoles) of 3'-O-ethyl-5'-O-triphenylmethyl-2'-deoxy-5-iodouridine was added to the reaction mixture. The mixture was stirred at 60°C for 5 hours. The solvent was

removed from the reaction mixture by distillation under reduced pressure, and the residue was extracted with methylene chloride. The solvent was distilled off from the extract under reduced pressure after which 25 ml of 2% hydrochloric acid-methanol was added to the residue and the mixture was stirred at room temperature for 3 hours. After the reaction mixture was neutralized with triethylamine, the solvent was distilled off under reduced pressure. Water was added to the residue and the mixture was extracted with methylene chloride. After the extract was washed with water, the solvent was distilled off under reduced pressure. After addition of methanol to the residue, the precipitated crystals were separated by filtration, the mother liquor was concentrated under reduced pressure, chloroform was added to the concentrate and the precipitated crystals were collected by filtration, giving 0.61 g of 3'-O-ethyl-2'-deoxy-5-trifluoromethyluridine having a melting point of 181 to 183°C (52% yield).

Example 1

Preparation of 3'-O-ethyl-5'-O-triphenylmethyl-2'-deoxy-5-trifluoromethyluridine

A 20 ml quantity of HMPA was added to 1.25 g (2 mmoles) of 3'-O-ethyl-5'-O-triphenylmethyl-2'-deoxy-5-iodouridine and 2.95 g (46 mmoles) of powdery copper.

Into the mixture was introduced 7.1 g (36 mmoles) of trifluoromethane iodide. The mixture was stirred in a sealed tube at 110°C for 20 hours. The reaction mixture was poured into ice water and the mixture was extracted with ether-ethyl acetate. The solvent was distilled off from the extract under reduced pressure. The residue was subjected to silica gel column chromatography, whereby 0.46 g of 3'-O-ethyl-5'-O-triphenylmethyl-2'-deoxy-5-trifluoromethyluridine was obtained by elution with chloroform/ethanol (30 : 1) (37% yield).

Nuclear magnetic resonance spectrum

(TMS internal standard, in DMSO-$d_6$)

δ ppm;

Approximately 12 (1H, broad, NH), 8.13 (1H, s, $H^6$), 7.5-7.1 (15H, m, aromatic H), 5.95 (1H, t, $H^{1'}$), 4.04 (2H, m, $H^{3'}$ and $H^{4'}$), 3.40 (2H, q, $-OC\underline{H_2}CH_3$), 3.27 (2H, m, $H^{5'}$), 2.37 (1H, m, $H^{2'}$), 1.07 (3H, t, $-OCH_2C\underline{H_3}$)

Example 2

Preparation of 3'-O-ethyl-5'-O-triphenylmethyl-2'-deoxy-5-trifluoromethyluridine

A 20 ml quantity of dimethylformamide/pyridine (3 : 1) was added to 1.25 g (2 mmoles) of 3'-O-ethyl-5'-O-triphenylmethyl-2'-deoxy-5-iodouridine and 1.27 g (20 mmoles) of powdery copper. Into the mixture was

introduced 8.9 g (60 mmoles) of trifluoromethane bromide and the mixture was stirred in a sealed tube at 140°C for 20 hours. The solvent was distilled off under reduced pressure and water was added to the residue after which the mixture was extracted with methylene chloride. The solvent was removed from the extract by distillation under reduced pressure. The residue was subjected to silica gel column chromatography, whereby 0.39 g of 3'-O-ethyl-5'-O-triphenylmethyl-2'-deoxy-5-trifluoromethyluridine was obtained by elution with ether/petroleum ether (1 : 1) (34% yield).

Example 3

Preparation of 3'-O-benzyl-5'-O-triphenylmethyl-2'-deoxy-5-trifluoromethyluridine

A 9.8 g (0.05 mole) quantity of trifluoromethane iodide was added to 6.4 g (0.1 mole) of powdery copper and 25 ml of HMPA. The mixture was stirred in a sealed tube at 120°C for 2.5 hours. The reaction mixture was filtered in a nitrogen gas atmosphere using sellaite. To the mother liquor was added 1.72 g (2.5 mmoles) of 3'-O-benzyl-5'-O-triphenylmethyl-2'-deoxy-5-iodouridine. The mixture was stirred at room temperature for 10 hours. The reaction mixture was poured into ice water and the mixture was extracted with ether-ethyl acetate. The solvent was distilled off from the extract under reduced pressure

after which the residue was recrystallized from methanol, giving 0.80 g of 3'-O-benzyl-5'-O-triphenylmethyl-2'-deoxy-5-trifluoromethyluridine having a melting point of 154 to 156°C (51% yield).

Nuclear magnetic resonance spectrum

(TMS internal standard, in DMSO-$d_6$)

$\delta$ ppm;

11.94 (1H, broad, NH), 8.09 (1H, s, $H^6$), 7.5 to 7.1 (20H, m, aromatic H), 6.04 (1H, t, $H^{1'}$), 4.46 (2H, s, $-OC\underline{H_2}-$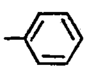 ), 4.13 (2H, m, $H^{3'}$ and $H^{4'}$), 3.24 (2H, m, $H^{5'}$), 2.40 (2H, m, $H^{2'}$)

Example 4

Preparation of 3'-O-benzyl-5'-O-triphenylmethyl-2'-deoxy-5-trifluoromethyluridine

To 3.0 g (0.047 mole) of powdery copper was added 24 ml of dimethylformamide/pyridine (3 : 1). Into the mixture was introduced 5.4 g (0.036 mole) of trifluoromethane bromide after which the mixture was stirred in a sealed tube at 140°C for 5 hours. After cooling, 824 mg (1.2 mmoles) of 3'-O-benzyl-5'-O-triphenylmethyl-2'-deoxy-5-iodouridine was added to the reaction mixture. The mixture was stirred at 60°C for 10 hours. The solvent was distilled off from the reaction mixture under reduced pressure and the residue was extracted wtih methylene chloride. The solvent was

evaporated off from the extract under reduced pressure after which the residue was recrystallized from methanol, giving 438 mg of 3'-O-benzyl-5'-O-triphenylmethyl-2'-deoxy-5-trifluoromethyluridine having a melting point of 154 to 155°C (58% yield).

Example 5

Preparation of 2'-deoxy-3'-O-ethyl-5-trifluoromethyl-5'-O-triphenylmethyluridine

HMPA (20 ml) was added to 1.15 g (2 mmoles) of 5-bromo-2'-deoxy-3'-O-ethyl-5'-O-triphenylmethyluridine and 2.95 g (46 mmoles) of powdery copper. Into the mixture was introduced 7.19 g (36 mmoles) of trifluoromethane iodide and the mixture was stirred in a sealed tube at 100°C for 24 hours. The reaction mixture was poured into ice water after which the mixture was extracted with ethyl acetate. The solvent was distilled off from the extract under reduced pressure, and the residue was subjected to silica gel column chromatography, whereby 0.40 g of 2'-deoxy-3'-O-ethyl-5-trifluoromethyl-5'-O-triphenylmethyluridine was obtained as a colorless oil from the chloroform eluate. The oil thus obtained was found identical with the product obtained in Example 1 in terms of physico-chemical constant.

Example 6

Preparation of 3'-O-benzyl-2'-deoxy-5-trifluoromethyl-5'-O-triphenylmethyluridine

To 3.0 g (47 mmoles) of powdery copper was added 24 ml of dimethylformamide/pyridine (2 : 1). Into the mixture was introduced 5.4 g (36 mmoles) of trifluoromethane bromide. The mixture was stirred in a sealed tube at 140°C for 5 hours. After cooling, 767 mg (1.2 mmoles) of 3'-O-benzyl-5-bromo-2'-deoxy-5'-O-triphenylmethyluridine was added to the reaction mixture, followed by stirring at 80°C for 16 hours. The solvent was distilled off from the reaction mixture under reduced pressure, and the residue was extracted with ethyl acetate. The solvent was distilled off from the extract under reduced pressure and the residue was recrystallized from methanol, giving 484 mg of 3'-O-benzyl-2'-deoxy-5-trifluoromethyl-5'-O-triphenylmethyluridine having a melting point of 154 to 155°C (64% yield).

Claims:

1. A 2'-deoxy-5-trifluoromethyluridine derivative represented by the formula

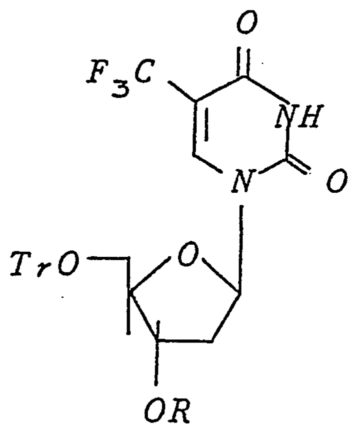

wherein Tr is triphenylmethyl and R is lower alkyl or benzyl.

2. A process for preparing a 2'-deoxy-5-trifluoromethyluridine derivative represented by the formula

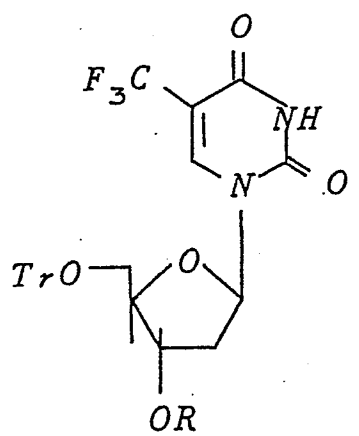

wherein Tr is triphenylmethyl and R is lower alkyl or

benzyl, the process comprising subjecting a 2'-deoxy-5-halogenouridine derivative represented by the formula

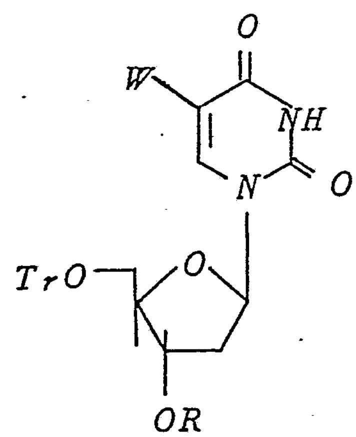

wherein W is iodine or bromine, and Tr and R are as defined above to trifluoromethylation reaction with a halogenotrifluoromethane in the presence of a metallic copper.

# INTERNATIONAL SEARCH REPORT

0252989

International Application No. PCT/JP86/00660

**I. CLASSIFICATION OF SUBJECT MATTER** (if several classification symbols apply, indicate all) [3]

According to International Patent Classification (IPC) or to both National Classification and IPC

Int.$Cl^4$ C07H19/073

**II. FIELDS SEARCHED**

Minimum Documentation Searched [4]

| Classification System | Classification Symbols |
| --- | --- |
| IPC | C07H19/073 |

Documentation Searched other than Minimum Documentation to the Extent that such Documents are Included in the Fields Searched [5]

**III. DOCUMENTS CONSIDERED TO BE RELEVANT** [14]

| Category* | Citation of Document, [16] with indication, where appropriate, of the relevant passages [17] | Relevant to Claim No. [18] |
| --- | --- | --- |
| A | JP, A, 60-61593 (Taiho Pharmaceutical Co., Ltd.) 9 April 1985 (09. 04. 85) P.3, left column, line 1 to p.4, left column, 7th line from the bottom & EP, A, 129,984 | 1-2 |
| A | JP, A, 57-88194 (Beecham Group Ltd.) 1 June 1982 (01. 06. 82) P.3, left column, line 8 to p.3, right column, 5th line from the bottom & EP, A, 49,110 & US, A, 4,383,990 | 1-2 |
| A | Journal of the American Chemical Society, Vol.84, No.18 (1962), Charles Heidelberger et al. [Synthesis of 5-Trifluoromethyluracil and 5-Trifluoromethyl-2'-Deoxy uridine] P.3597-3598 | 1-2 |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search [2] | Date of Mailing of this International Search Report [2] |
| --- | --- |
| March 24, 1987 (24. 03. 87) | April 6, 1987 (06. 04. 87) |

| International Searching Authority [1] | Signature of Authorized Officer [20] |
| --- | --- |
| Japanese Patent Office | |